(19) Europäisches Patentamt European Patent Office Office européen des brevets

(11) **EP 3 100 672 A1**

(12) **EUROPEAN PATENT APPLICATION**
published in accordance with Art. 153(4) EPC

(43) Date of publication:
**07.12.2016 Bulletin 2016/49**

(51) Int Cl.:
***A61B 5/00*** (2006.01)

(21) Application number: **14881298.5**

(86) International application number:
**PCT/JP2014/082393**

(22) Date of filing: **08.12.2014**

(87) International publication number:
**WO 2015/114950 (06.08.2015 Gazette 2015/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.01.2014 JP 2014015411**

(71) Applicant: **Konica Minolta, Inc.**
**Tokyo 100-7015 (JP)**

(72) Inventors:
• **MATSUDA, Shinya**
**Tokyo 100-7015 (JP)**
• **KURODA, Eiji**
**Tokyo 100-7015 (JP)**
• **MOTOKUI, Yasuyuki**
**Tokyo 100-7015 (JP)**

(74) Representative: **Gille Hrabal**
**Brucknerstrasse 20**
**40593 Düsseldorf (DE)**

(54) **ORGAN IMAGE CAPTURING DEVICE**

(57) An organ image capturing device (1) comprises an imaging unit (3) to image the tongue of a living body, and a computing unit (16). The computing unit (16) calculates a numerical value indicating the state of at least one of a plurality of indices pertaining to health by way of computations using chromaticity values that are obtained from captured image data of the tongue acquired by the imaging unit (3).

FIG.6

RELATIONSHIP BETWEEN B PROPORTION IN UPPER REGION AND SYSTOLIC /DIASTOLIC BLOOD PRESSURES

$y = -944.54x + 414.69$

$y = -1033x + 379.35$

BLOOD PRESSURE (mmHg)

B PROPORTION IN UPPER REGION

◆ SYSTOLIC BLOOD PRESSURE
□ DIASTOLIC BLOOD PRESSURE

## Description

## Technical Field

The present invention relates to an organ imaging device used to image the tongue as an organ of a living body to extract information needed for a healthiness checkup.

## Background Art

Conventionally known methods for grasping human health condition include biochemical examination as part of a medical checkup, a measurement of physical strength, and a subjective-symptom report via a medical questionnaire.

However, the medical checkup and the measurement of physical strength, which are costly and take time, are not appropriate as a method for monitoring daily variation in physical condition. As for the medical questionnaire, in which subjective symptoms are reported as answers to questions, descriptions of such subjective symptoms greatly vary from person to person, and thus it is difficult to properly conduct comparison of such descriptions with past records or with data of another person.

Against the background discussed above, there has recently been proposed a system in which an image of the tongue is taken with a digital camera to examine healthiness by using a feature value of the taken image. For example, according to the disclosure of Patent Literature 1 listed below, it is possible to make an easy and objective judgment on the general healthiness of the owner of the imaged tongue by calculating one kind of output information (specifically, Mahalanobis' Distance) from many kinds of feature values obtained from the taken image of the tongue. Such a system of making a general judgment on healthiness by using Mahalanobis' Distance is proposed also in, for example, Patent Literature 2 listed below.

## Citation List

## Patent Literature

Patent Literature 1: Japanese Patent No. 4487535 (please refer to claim 1, paragraphs [0006], [0018], and [0096], etc.)
Patent Literature 2: Japanese Patent No. 4649965 (please refer to claim 1, paragraph [0008], etc.)

## Summary of Invention

## Technical Problem

However, the systems disclosed in Patent Literatures 1 and 2 above are intended for making a general (a single) judgment on healthiness from a taken image of the tongue, and are not intended for obtaining information regarding a plurality of healthiness indices (such as blood pressure and body temperature). Accordingly, the systems do not help users to minutely grasp their own physical conditions and symptoms, making it difficult for the user to decide on specific measures to take against their physical conditions and symptoms, which may be buying a nonprescription drug suitable for their physical conditions, consulting a medical institution suitable for their physical conditions and symptoms, etc.

The present invention has been made to solve this problem, and an object thereof is to provide an organ imaging device that makes it easier for a user to monitor daily variation in his or her physical condition, to compare current information with past records or with information of another person, and to make a decision on specific measures suitable according to his or her physical condition and symptom.

## Solution to Problem

According to one aspect of the present invention, an organ imaging device includes an imager which images a tongue of a living body, and a processor which calculates a numerical value indicating condition regarding at least one of a plurality of healthiness indices by using a chromaticity value obtained from data of a taken image of the tongue acquired by the imager, and outputs the numerical value.

## Advantageous Effects of Invention

According to the above configuration, based on the numerical value calculated with respect to at least one of the plurality of healthiness indices, it becomes easy for the user to monitor daily variation in his or her physical condition, to conduct comparison with past records or with information of another person, and to make a decision on specific measures suitable according to his or her physical condition and symptom.

## Brief Description of the Drawings

FIG. 1 is a perspective view of an exterior of an organ imaging device according to an embodiment of the present invention;
FIG. 2 is a block diagram showing an outline configuration of the organ imaging device;
FIG. 3 is an explanatory diagram illustrating a positional relationship between an illuminator and an imager of the organ imaging device with respect to an imaging object;
FIG. 4 is an explanatory diagram illustrating an image of a tongue taken by the imager, an edge extraction filter, and a contour line of the tongue extracted from the taken image by using the edge extracting filter;

FIG. 5 is an explanatory diagram illustrating a positional relationship between the contour line of the tongue and three regions chromaticity values of which are to be calculated;

FIG. 6 is a graph illustrating a relationship between B proportion in an upper region of the taken image of the tongue and systolic/diastolic blood pressures;

FIG. 7 is a graph illustrating a relationship between R proportion in the upper region of the taken image of the tongue and the condition of constipation;

FIG. 8 is a graph illustrating a relationship between G proportion in a center region of the taken image of the tongue and the condition of diarrhea;

FIG. 9 is a graph illustrating a relationship between G proportion in the center region of the taken image of the tongue and general physical weariness;

FIG. 10 is a graph illustrating a relationship between B proportion in the center region of the taken image of the tongue and the condition of under-eye dark circles;

FIG. 11 is a graph illustrating a relationship between R proportion in a lower region of the taken image of the tongue and body temperature;

FIG. 12 is a graph illustrating a relationship between G proportion in the lower region of the taken image of the tongue and sore throat;

FIG. 13 is a graph illustrating a relationship between the G proportion in the lower region of the taken image of the tongue and the severity of leg cramp;

FIG. 14 is a flowchart illustrating an operation flow of the organ imaging device;

FIG. 15 is a graph illustrating a relationship between B/R ratio in the upper region of the taken image of the tongue and systolic/diastolic blood pressures; and

FIG. 16 is a graph illustrating a relationship between B/(R+ G) ratio in the upper region of the taken image of the tongue and systolic/diastolic blood pressures.

**Description of Embodiments**

Following hereinbelow is a description of an embodiment of the present invention, with reference being made to the accompanying drawings. In this specification, when a numerical value range is indicated as A to B, the lower limit A and the upper limit B are both included in the numerical value range.

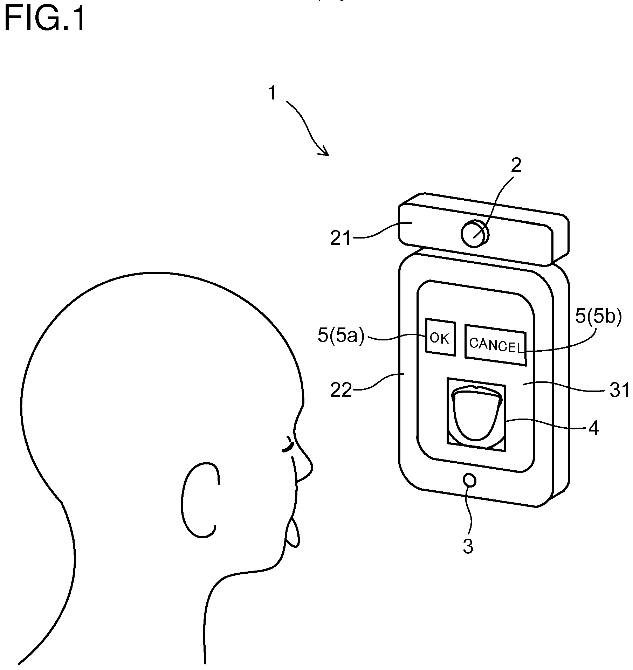

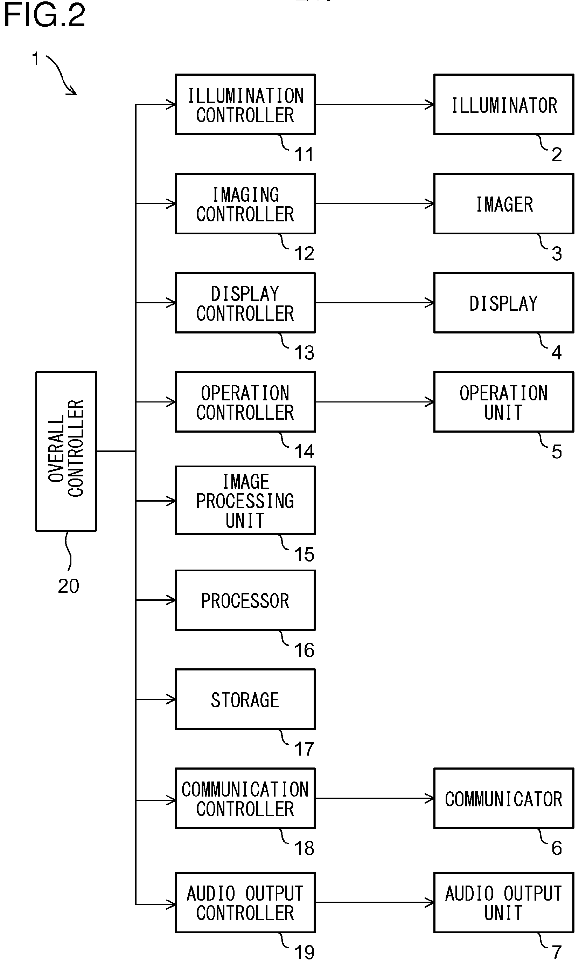

**Overall Configuration of Organ Imaging Device:** FIG. 1 is a perspective view of an exterior of an organ imaging device 1 according to the present embodiment, and FIG. 2 is a block diagram illustrating an outline configuration of the organ imaging device 1. The organ imaging device 1 images the tongue as an organ of a living body to extract, from the taken image, information necessary for a healthiness checkup.

The organ imaging device 1 includes an illuminator 2, an imager 3, a display 4, an operation unit 5, a communicator 6, and an audio output unit 7. The illuminator 2 is provided at a housing 21, and the blocks other than the illuminator 2 (e.g., the imager 3, the display 4, the operation unit 5, the communicator 6, and the audio output unit 7) are provided at a housing 22. The housing 21 and the housing 22 are coupled together so as to be rotatable relative to each other, but the rotatability is not necessarily indispensable, and one may be completely fixed to the other. The illuminator 2 and the other blocks may be provided at a single housing. Further, the organ imaging device 1 may be configured as a multifunction portable information terminal.

The illuminator 2 is configured as a light that illuminates an imaging object from above. Used as a light source of the illuminator 2 is one that emits light of daylight color, such as a xenon lamp, for improved color reproduction. The brightness of the light source varies depending on the sensitivity of the imager 3 and the distance to the imaging object; the brightness can be, for example, such as to provide an illumination of 1000 to 10000 lx at the imaging object. The illuminator 2 includes, in addition to the light source, a lighting circuit and a dimming circuit, and is controlled according to instructions from an illumination controller 11 so as to be turned on/off, and dimmed.

The imager 3 images an organ (here, the tongue) of a living body to acquire an image of it under illumination provided by the illuminator 2, and includes an imaging lens and an area sensor (an image sensor). The aperture of the imaging lens (the fastness of the lens), the shutter speed, and the focal length are so set that the imaging object is in focus over its entire area. For example, the f-number can be set at 16, the shutter speed can be set at 1/120 seconds, and the focal length can be set at 20 mm.

The area sensor is configured with an image sensor such as a CCD (charge-coupled device) image sensor or a CMOS (complementary metal oxide semiconductor) image sensor, for example, and its sensitivity, resolution, etc. are so set that the color and the shape of the imaging object can be detected satisfactorily. For example, the sensitivity can be set at 60 db, and the resolution can be set at 10 megapixels.

The imaging performed by the imager 3 is controlled by an imaging controller 12. The imager 3 further includes, in addition to the imaging lens and the area sensor, a focusing mechanism, an aperture mechanism, a drive circuit, an A/D conversion circuit, etc., of which none is illustrated, and is controlled according to instructions from the imaging controller 12 in terms of focus, aperture, A/D conversion, etc. The imager 3 acquires, as the data of a taken image, data having, for example, eight bits, representing a value from 0 to 255, for each of red (R), green (G), and blue (B).

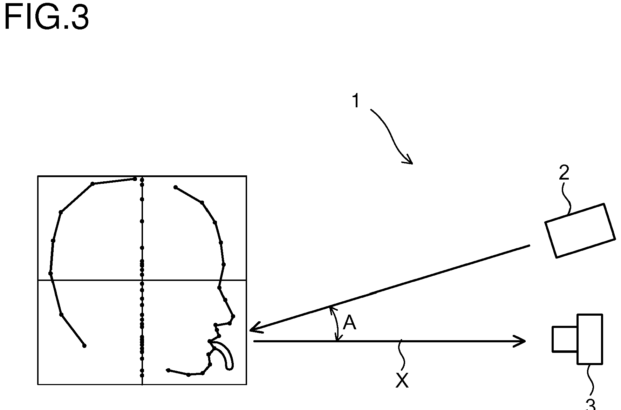

FIG. 3 is a diagram illustrating a positional relationship between the illuminator 2 and the imager 3 with respect to an imaging object (such as the tongue or the face). As shown in the figure, the imager 3 is arranged straight in front of the imaging object. The illuminator 2 is so arranged as to illuminate the imaging object, for example, at an angle A of 0° to 45° relative to an imaging optical axis X of the imager 3, which passes through the imaging object. The imaging optical axis X denotes the optical axis of the imaging lens provided in the imager 3.

When illumination is applied at a large angle A, the shadow of the upper lip reduces the area over which the tongue can be imaged. Conversely, when illumination is applied at a small angle A, specular reflection causes severe color clipping. With these taken into consideration, a preferred range of the angle A for illumination is from 15° to 30°.

The display 4 includes a liquid crystal panel, a backlight, a lighting circuit, and a control circuit, of which none is illustrated, and the display 4 displays an image acquired by the imaging performed by the imager 3, and information calculated by and outputted from a processor 16 which will be described later. The display 4 can also display information (e.g., the result of a diagnosis made at an external medical institution based on information transmitted thereto) acquired from outside via the communicator 6. The display of information of various kinds performed at the display 4 is controlled by a display controller 13.

The operation unit 5 is an input unit via which to instruct the imager 3 to perform imaging, and includes an OK button (TAKE IMAGE button) 5a and a CANCEL button 5b. In the present embodiment, the display 4 and the operation unit 5 are constituted by a single touch panel display device 31, and separate display areas are provided on the touch panel display device 31, one area for the display 4 and the other area for the operation unit 5. The display of the operation unit 5 on the touch panel display device 31 is controlled by an operation controller 14. Here, the operation unit 5 may be configured as any other input device than the touch panel display device 31 (the operation unit 5 may be provided anywhere else than inside the display area of the touch panel display device 31).

The communicator 6 is an interface for transmitting data of images obtained by the imager 3 and information calculated and outputted from the later-described processor 16 to outside via a communication network (which may be wired or wireless), and for receiving information from outside. The transmission and reception of information performed at the communicator 6 is controlled by a communication controller 18.

The audio output unit 7 outputs a variety of information in the form of sound, and is configured with a speaker, for example. The information outputted in the form of sound includes a result of calculation performed by the processor 16 (a numerical value of each healthiness index). The output of sound from the audio output unit 7 is controlled by an audio output controller 19.

The organ imaging device 1 further includes the illumination controller 11, the imaging controller 12, the display controller 13, the operation controller 14, the image processing unit 15, the processor 16, a storage 17, the communication controller 18, the audio output controller 19, and an overall controller 20 which controls all these blocks. The illumination controller 11, the imaging controller 12, the display controller 13, the operation controller 14, the communication controller 18, and the audio output controller 19 control the illuminator 2, the imager 3, the display 4, the operation unit 5, the communicator 6, and the audio output unit 7, respectively, as described above. The overall controller 20 is configured with a central processing unit (CPU), for example. The illumination controller 11, the imaging controller 12, the display controller 13, the operation controller 14, the communication controller 18 and the audio output controller 19 may be integrally configured with the overall controller 20 (in a single CPU, for example).

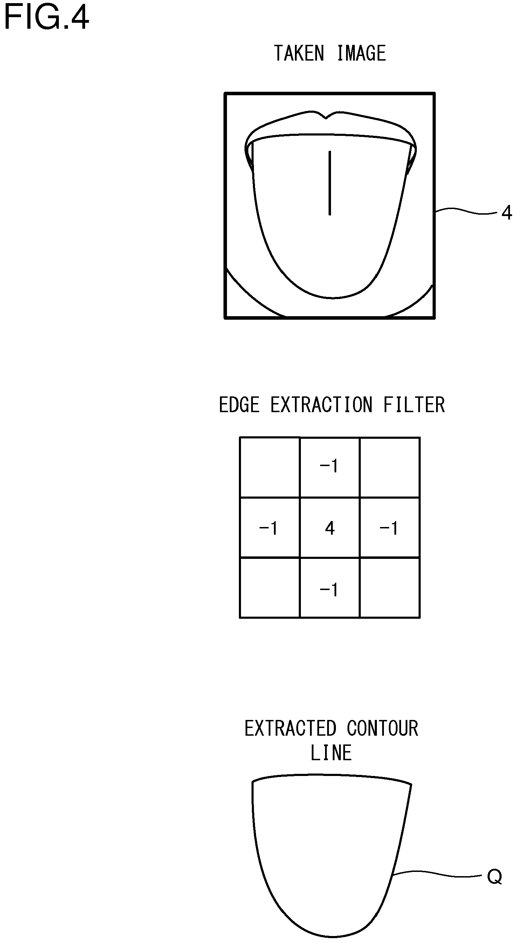

The image processing unit 15 has a function of extracting a contour line of an organ from an image acquired by the imager 3. The extraction of the contour line of an organ can be performed by extracting a luminance edge (a part where image brightness changes sharply) in the taken image, and the extraction of a luminance edge can be performed, for example, by using an edge extraction filter as shown in FIG. 4. An edge extraction filter is a filter that gives weights to pixels near a pixel of interest when performing first-order differentiation (when calculating differences in image data between neighboring pixels).

By using such an edge extraction filter, for example, with respect to the green image data of each pixel in the taken image, differences in image data are calculated between the pixel of interest and neighboring pixels, and such pixels as yield differences exceeding a predetermined threshold value are extracted; in this way, pixels that constitute a luminance edge can be extracted. Around the tongue, its shadow produces luminance differences; thus, by extracting pixels that constitute a luminance edge in the manner described above, it is possible to extract the contour line of the tongue. Here, green image data is used in the calculation because it has the greatest influence on luminance; however, red or blue image data may be used instead.

In Oriental medicine, a white patch of coating seen in a center part of the tongue is called "tongue coating," and its color is called "tongue coating color." On the other hand, the color of the rest, the red part, of the tongue is called "tongue color."

The storage 17 is a memory that stores therein data of images acquired by the imager 3, information acquired by the image processing unit 15, data calculated by the processor 16, information received from the outside, etc., and programs for operating the various controllers described above.

The processor 16 obtains a chromaticity value from data of a taken image of a tongue acquired by the imager 3, performs calculation by using the obtained chromaticity value to calculate a numerical value indicating condition regarding at least one of a plurality of healthiness indices (judgment items), and outputs the numerical value. The plurality of healthiness indices include, for example, systolic /diastolic blood pressures, the condition of constipation, the condition of diarrhea, the condition of physical weariness caused by fatigue (presence/absence of general physical weariness), the condition of blood circulation, body temperature, the condition of bronchi, and the condition of muscle fatigue. The numerical value calculated by the processor 16 is displayed at the display 4, or outputted in the form of sound from the audio output unit 7. Thus, it can be said that the display 4 and the audio output unit 7 constitute an output unit which displays, or outputs in the form of sound, the numerical value calculated by and outputted from the processor 16. Data of the numerical value calculated by the processor 16 may be fed to an external terminal device via the communicator 6.

Here, the chromaticity value used to calculate the numerical value is obtained in any of three upper, center, and lower regions in the center part of the taken image of the tongue in the left/right direction that most appropriately corresponds to each of the indices. Hereinbelow, descriptions will first be given of these three regions.

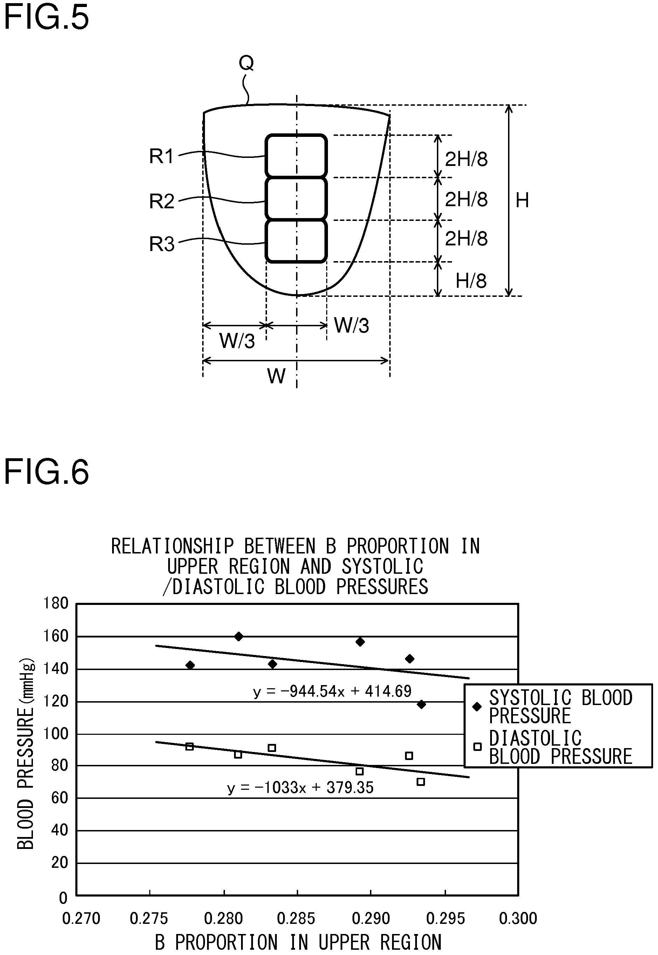

**Chromaticity Value Calculation Region:** FIG. 5 illustrates a positional relationship between a contour line Q of a tongue and three regions R1, R2, and R3, chromaticity values of which are to be calculated. The regions R1, R2, and R3 respectively correspond to an upper region, a center region, and a lower region in a center part of the taken image of the tongue in the left/right direction. The regions R1, R2, and R3 are each sized and positioned in the positional relationship as shown in the figure, where H represents a top-to-bottom length of the contour line Q extracted by the image processing unit 15, and W represents a left/right direction length (width) of the contour line Q. Note that the sizes of the regions R1, R2, and R3 in the figure are merely an example, and are not limited to this example. These three regions R1, R2, and R3 are set as regions chromaticity values of which are to be calculated for the following reason.

Various diagnosis items are proposed for the tongue diagnosis used in Oriental medicine (traditional Chinese (*Kampo*) medicine). Four of such proposed diagnosis items are mainly used, namely, the color and the shape (thickness) of the tongue and the color and the shape (thickness) of the tongue coating. The tongue coating is formed of cornified cells of papillae of the tongue mucous membrane; it is observed in upper and center parts of a band-shaped region extending from top to bottom of the center of the tongue in the left/right direction, and a large amount of tongue coating is observed particularly in the upper part. Thus, variation in color of the tongue coating appears as variation in chromaticity of the upper region R1. That is, the tongue coating takes on a white to brown color depending on the amount of cornified cells in the tongue coating, and thus, in the upper region R1, mainly the G component increases or decreases in the RGB image data.

On the other hand, the color of the tongue is generally judged based on right and left end parts or a lower part of the tongue where there is no tongue coating. At the right and left end parts of the tongue, there tends to occur a shade of color because illumination light is incident on irregularities of the tongue surface at different angles. Thus, it is desirable to judge the color of the tongue by using image data of the lower region R3. The color of the tongue reflects the color of blood, and thus, in the region R3, mainly an amount of R or B component increases or decreases among RGB image data. That is, when the color of the tongue changes, the chromaticity of the lower region R3 also changes.

The thickness of the tongue coating can be detected by using difference in color from the color of the tongue beneath the tongue coating. That is, in the center of the taken image of the tongue in the left/right direction, if the color of the center part between the upper and lower parts is close to that of the upper part, it means that the tongue coating is thick, and on the other hand, if the color of the center part is close to that of the lower part, it means that the tongue coating is thin. Thus, variation in thickness of the tongue coating appears as variation in chromaticity of the center region R2. That is, as the tongue coating grows thicker, the color of the tongue coating changes from the red color of the tongue itself to the white color of the tongue coating itself; thus, in the region R2, mainly the R or G component decreases/increases in the RGP image data.

Thus, by using the chromaticity value of at least any of the regions R1 to R3, it is possible to reproduce the diagnostic method (the tongue diagnosis) where judgment of healthiness is performed by judging the color or the shape of the tongue or of the tongue coating from the chromaticity value, which is practiced in Oriental medicine.

At this time, by considering, as the chromaticity value, the proportion of R image data (R proportion), the proportion of G image data (G proportion), or the proportion of B image data (B proportion) with respect to a sum of the RGB image data as reference data in each of the regions R1 to R3, it is possible to securely reduce influence of the brightness under which the imaging of the tongue is performed. That is, although at least any of the RGB image data increases if the imaging is performed in a bright environment, by using the proportion of the image data of a desired color with respect to the reference data, namely the sum, as the chromaticity value, it is possible to reduce variation in chromaticity value and in calculated numerical value of each of the indices caused by the brightness of the environment.

The R proportion in each of the regions R1 to R3 can be defined as an average of R proportions in all the pixels of each of the regions R1 to R3. Likewise, the G proportion and the B proportion in each of the regions R1 to R3 can be defined as an average of G proportions and an average of B proportions, respectively, in each of the regions R1 to R3.

Here, the chromaticity value may be a numerical value obtained by using various color systems other than the RGB color system (for example, Yxy, Lab, etc.), and in whichever case, it is possible to reproduce the diagnostic method (tongue diagnosis) practiced in Oriental medicine.

**Quantification of Index:** Next, a description will be given of a specific example of quantifying the plurality of indices regarding healthiness performed by the processor 16 through calculation by using the chromaticity value. Here, the R proportion, the G proportion, and the B proportion in the following description are, as described in the above description, the proportions, with respect to the sum of RGB image data, of the R image data (R/(R+G+B)), the G image data (G/(R+G+B)), and the B image data (B/(R+G+B)), respectively.

The inventors of the present invention conducted a survey on correlation between feature values extracted from taken images of tongues and examination items regarding healthiness. Feature values extracted from each of the taken images of tongues are the R proportion, the G proportion, and the B proportion of each of the above-described three regions R1 to R3 (that is, a total of nine feature values). The examination items are 35 items constituted by 30 items including living condition and physical condition, and 5 items including body temperature, pulse rate, and blood pressure. The survey was conducted in the following manner: For the former 30 items, information was collected from individual examinees as answers to questions, and measurements were conducted for the latter 5 items. As a result, the inventors found correlation between some of these items.

**Blood Pressure:** FIG. 6 is a graph illustrating a relationship between the B proportion in the upper region (the region R1) of the taken image of the tongue and systolic/diastolic blood pressures. From this figure, which shows that the B proportion in the upper region decreases as whichever of the systolic blood pressure and the diastolic blood pressure rises, it is clear that there is a correlation between them. Thus, if a regression formula (an approximate formula) representing the relationship between them can be obtained, the systolic/diastolic blood pressures can be estimated from the B proportion in the upper region by using the regression formula.

Specifically, the following formulae were obtained as regression formulae from the scattered data shown in FIG. 6 by a least squares method:

$$\text{Systolic blood pressure: } y = -950x + 400$$

$$\text{Diastolic blood pressure: } y = -1000x + 400$$

where variable x represents the B proportion in the upper region, and variable y represents the blood pressure (mmHg).

Thus, the processor 16 can calculate numerical values of the systolic/diastolic blood pressures as healthiness indices by obtaining the B proportion in the upper region from the data (RGB image data) of the taken image of the tongue and substituting the B proportion into these regression formulae.

Note that it has been found that the relationship between the B proportion in the upper region and the systolic/diastolic blood pressures differs from person to person. Such individual differences can be adjusted for higher calculation accuracy by conducting data sampling for a period of time to obtain formulae (regression formulae) representing the relationship between them, and by calculating the systolic/diastolic blood pressures using the thereby obtained regression formulae.

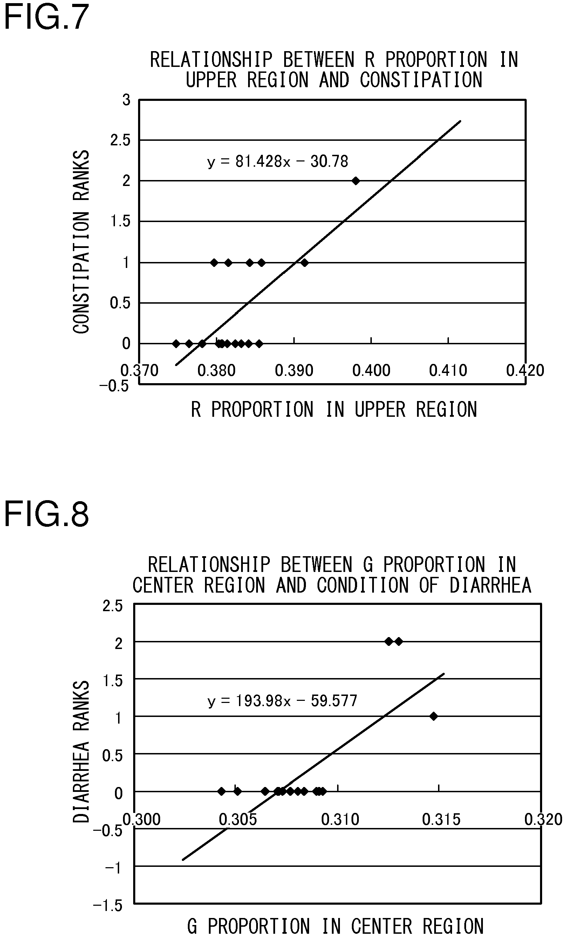

**Constipation:** FIG. 7 is a graph illustrating a relationship between the R proportion in the upper region (the region R1) of the taken image of the tongue and the condition of constipation. The condition of constipation is classified into four ranks (0 to 3) that quantify subjective symptoms of constipation. Constipation shall be increasingly severer from rank 0 to rank 3. From this figure, which shows that the R proportion in the upper region increases as the condition of constipation becomes severer, it is clear that there is a correlation between them. Thus, if a regression formula representing the relationship between them can be obtained, the condition of constipation can be estimated from the R proportion in the upper region by using the regression formula.

Specifically, the following formula was obtained as a regression formula from the scattered data shown in FIG. 7 by the least squares method:

$$Y = 80x - 30$$

where variable x represents the R proportion in the upper region, and variable y represents the value (the rank) indicating the condition of constipation.

Thus, the processor 16 can calculate the value (the rank) indicating the condition of constipation as a healthiness index by obtaining the R proportion in the upper region from the data of the taken image of the tongue and substituting the R proportion into the regression formula. Individual differences are adjusted here, too, as in the calculation of the systolic/diastolic blood pressures.

**Diarrhea:** FIG. 8 is a graph illustrating a relationship between the G proportion in the center region (the region R2) of the taken image of the tongue and the condition of diarrhea. The condition of diarrhea is classified into four ranks (0 to 3) that quantify subjective symptoms of diarrhea. Here, the condition of diarrhea shall be increasingly severer from rank 0 to rank 3. From this figure, which shows that the G proportion in the center region increases as the condition of diarrhea becomes severer, it is clear that there is a correlation between them. Thus, if a regression formula representing the relationship between them can be obtained, the condition of diarrhea can be estimated from the G proportion in the center region by using the regression formula.

Specifically, the following formula was obtained as a regression formula from the scattered data shown in FIG. 8 by the least squares method:

$$y = 190x - 60$$

where variable x represents the G proportion in the center region, and variable y represents the value (the rank) indicating the condition of diarrhea.

Thus, the processor 16 can calculate the value (the rank) indicating the condition of diarrhea as a healthiness index by obtaining the G proportion in the center region from the data of the taken image of the tongue and substituting the G proportion into the regression formula. Individual differences are adjusted here, too, as in the calculation of the systolic/diastolic blood pressures.

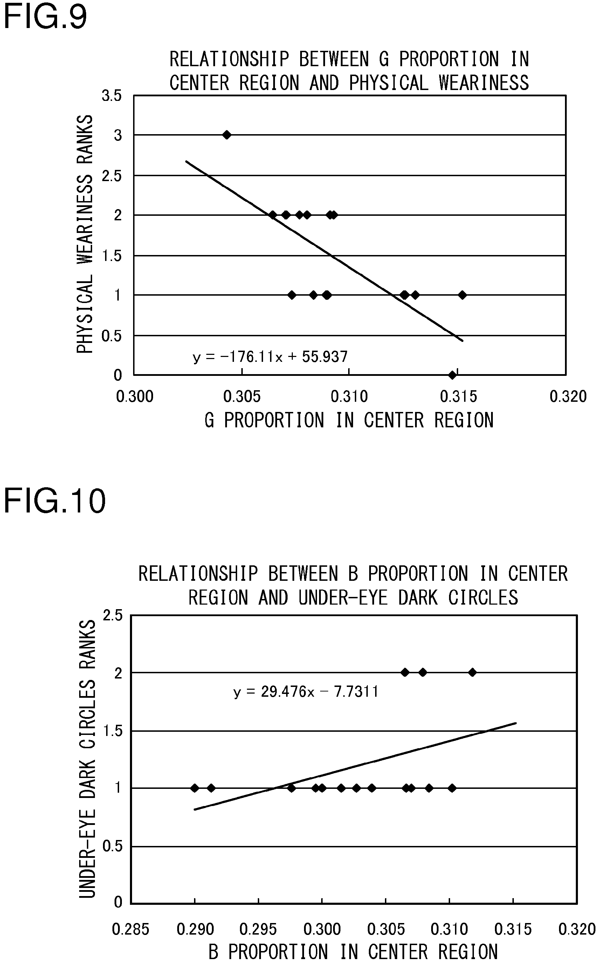

**Physical Weariness Caused by Fatigue:** FIG. 9 is a graph illustrating a relationship between the G proportion in the center region (the region R2) of the taken image of the tongue and general physical weariness. Physical weariness is classified into four ranks (0 to 3) that quantify subjective symptoms of physical weariness. Here, physical weariness shall increase (increasingly worsen) from rank 0 to rank 3. From this figure, which shows that the G proportion in the center region decreases as physical weariness increases, it is clear that there is a correlation between them. Thus, if a regression formula representing the relationship between them can be obtained, the condition of physical weariness caused by fatigue can be estimated from the G proportion in the center region by using the regression formula.

Specifically, the following formula was obtained as a regression formula from the scattered data shown in FIG. 9 by the least squares method:

$$y = -180x + 60$$

where variable x represents the G proportion in the center region, and variable y represents the value (the rank) indicating the physical weariness.

Thus, the processor 16 can calculate the value (the rank) indicating the condition of physical weariness caused by fatigue as a healthiness index by obtaining the G proportion in the center region from the data of the taken image of the tongue and substituting the G proportion into the regression formula. Individual differences are adjusted here, too, as in the calculation of the systolic/diastolic blood pressures.

**Blood Circulation:** FIG. 10 is a graph illustrating a relationship between the B proportion in the center region (the region R2) of the taken image of the tongue and the condition of under-eye dark circles. The condition of under-eye dark circles is classified into four ranks (0 to 3) that quantify degrees of under-eye dark circles. Here, under-eye dark circles shall appear increasingly darker and blood circulation shall be increasingly poorer from rank 0 to rank 3. From this figure, which shows that the B proportion in the center region increases as the under-eye dark circles become darker and the blood circulation becomes poorer, it is clear that there is a correlation between them. Thus, if a regression formula representing the relationship between them can be obtained, the condition of blood circulation can be estimated from the B proportion in the center region by using the regression formula.

Specifically, the following formula was obtained as a regression formula from the scattered data shown in FIG. 10 by the least squares method:

$$y = 30x - 10$$

where variable x represents the B proportion in the center region, and variable y represents the value (the rank) indicating the condition of under-eye dark circles.

Thus, the processor 16 can calculate the value (the rank) indicating the condition of blood circulation as a healthiness index by obtaining the B proportion in the center region from the data of the taken image of the tongue and substituting the B proportion into the regression formula. Individual differences are adjusted here, too, as in the calculation of the systolic/diastolic blood pressures.

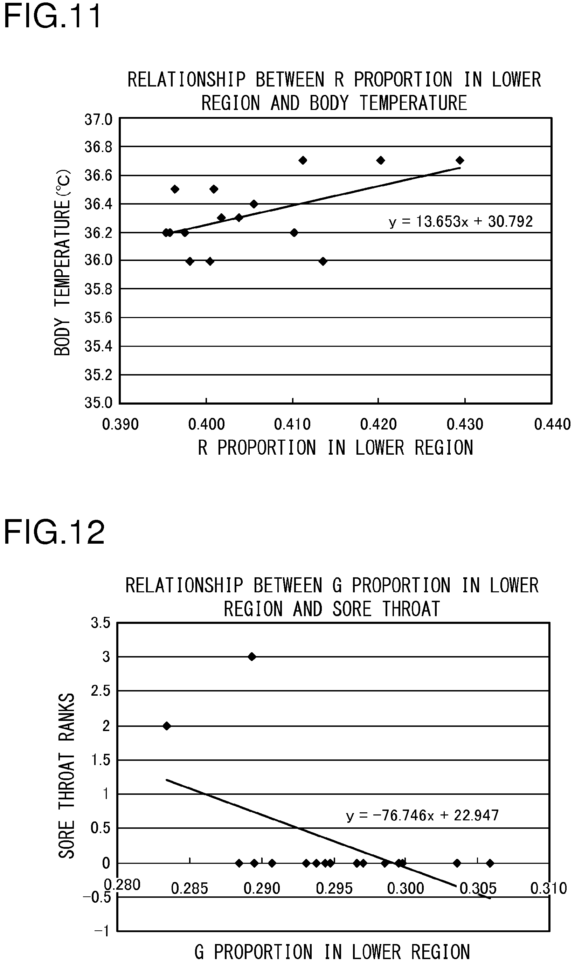

**Body Temperature:** FIG. 11 is a graph illustrating a relationship between the R proportion in the lower region (the region R3) of the taken image of the tongue and body temperature. From this figure, which shows that the R proportion in the lower region increases as the body temperature rises, it is clear that there is a correlation between them. Thus, if a regression formula representing the relationship between them can be obtained, the body temperature can be estimated from the R proportion in the lower region by using the regression formula.

Specifically, the following formula was obtained as a regression formula from the scattered data shown in FIG. 11 by the least squares method:

$$y = 15x + 30$$

where variable x represents the R proportion in the lower region, and variable y represents the body temperature (°C).

Thus, the processor 16 can calculate the numerical value of the body temperature as a healthiness index by obtaining the R proportion in the lower region from the data of a taken image of a tongue and substituting the R proportion into the regression formula. Individual differences are adjusted here, too, as in the calculation of the systolic/diastolic blood pressures.

**Condition of Bronchi:** FIG. 12 is a graph illustrating a relationship between the G proportion in the lower region (the region R3) of the taken image of the tongue and sore throat. The condition of sore throat is classified into four ranks (0 to 3) that quantify subjective symptoms of sore throat. Here, the condition of sore throat shall be increasingly severer and the condition of bronchi shall be increasingly poorer from rank 0 to rank 3. From this figure, which shows that the G proportion in the lower region decreases as the condition of sore throat becomes severer and the condition of bronchi becomes poorer, it is clear that there is a correlation between them. Thus, if a regression formula representing the relationship between them can be obtained, the condition of bronchi can be estimated from the G proportion in the lower region by using the regression formula.

Specifically, the following formula was obtained as a regression formula from the scattered data shown in FIG. 12 by the least squares method:

$$y = -80x + 20$$

where variable x represents the G proportion in the lower region, and variable y represents the rank indicating the condition of sore throat.

Thus, the processor 16 can calculate a numerical value that indicates the condition of bronchi as a healthiness index by obtaining the G proportion in the lower region from the data of a taken image of a tongue and substituting the G proportion into the regression formula. Individual differences are adjusted here, too, as in the calculation of the systolic/diastolic blood pressures.

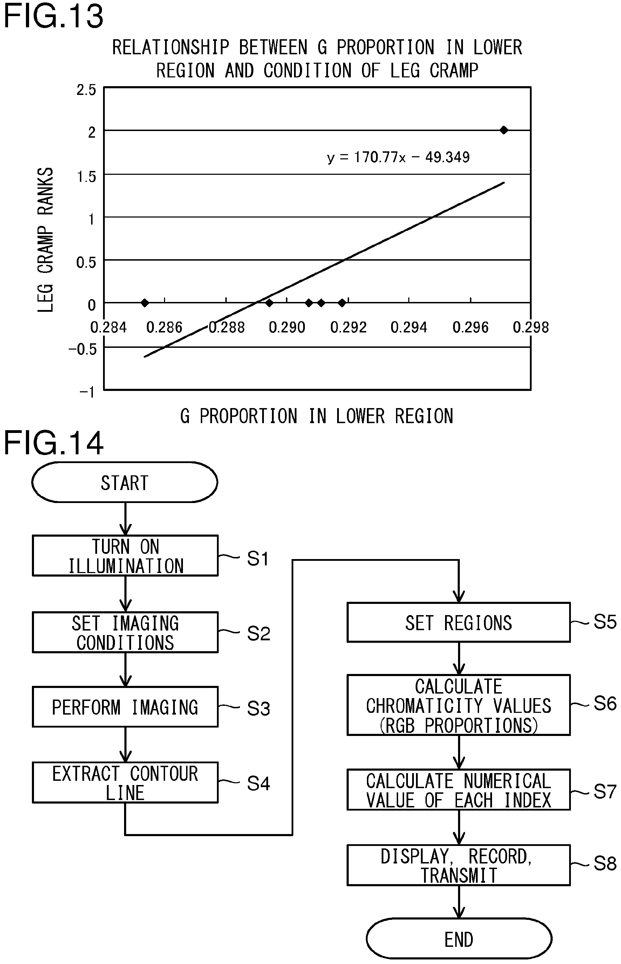

**Condition of Muscle Fatigue:** FIG. 13 is a graph illustrating a relationship between the G proportion in the lower region (the region R3) of the taken image of the tongue and the severity of leg cramp (the degree of contraction·convulsion of a leg muscle). The condition of leg cramp is classified into four ranks (0 to 3) that quantify subjective symptoms of leg cramp. Here, leg cramp shall be increasingly severer and muscle fatigue shall increase from rank 0 to rank 3. From this figure, which shows that the G proportion in the lower region increases as leg cramp becomes severer and muscle fatigue increases, it is clear that there is a correlation between them. Thus, if a regression formula representing the relationship between them can be obtained, the condition of muscle fatigue can be estimated from the G proportion in the lower region by using the regression formula.

Specifically, the following formula was obtained as a regression formula from the scattered data shown in FIG. 13 by the least squares method:

$$y = 170x - 50$$

where variable x represents the G proportion in the lower region , and variable y represents the rank indicating the condition of leg cramp.

Thus, the processor 16 can calculate a numerical value that indicates the condition of muscle fatigue as a healthiness index by obtaining the G proportion in the lower region from the data of a taken image of a tongue and substituting the G proportion into the regression formula. Individual differences are adjusted here, too, as in the calculation of the systolic/diastolic blood pressures.

**Control Flow:** FIG. 14 is a flowchart illustrating an operation flow in the organ imaging device 1 of the present embodiment. In the organ imaging device 1, in response to an imaging instruction received via the operation unit 5 or from an unillustrated input device, the illumination controller 11 turns on the illuminator 2 (S1), and sets conditions for imaging, such as illumination, etc. (S2). On completion of the setting of the conditions for imaging, the imaging controller 12 controls the imager 3 so as to perform imaging of a tongue as an imaging object (S3).

On completion of the imaging, the image processing unit 15 extracts a contour line Q of the tongue from its taken image (S4). Then, the processor 16 detects top, bottom, left, and right ends of the tongue from the extracted contour line Q, and, as illustrated in FIG. 5, the processor 16, with reference to the contour line Q, sets the three regions R1 to R3 chromaticity values of which are to be obtained (S5).

Next, the processor 16 obtains, from the data of the taken image of the tongue, chromaticity values of the regions R1 to R3, that is, the R proportion, the G proportion, and the B proportion in the regions R1 to R3 (S6), and then the processor 16 substitutes the thus obtained chromaticity values into regression formulae set in advance, and calculates numerical values each indicating the condition regarding a corresponding one of the plurality of healthiness indices (S7). The processor 16 is supposed to perform calculation with respect to at least one of the plurality of indices to obtain the numerical value indicating the regarding condition, and the processor 16 may calculate the numerical value with respect to only one index. The thus calculated numerical value, which is outputted from the processor 16 to be displayed on the display 4 or stored in the storage 17, is outputted from the audio output unit 7 in the form of sound, recorded at an unillustrated output device, or externally transmitted via the communicator 6, as necessary (S8).

As described above, in the present embodiment, instead of integrating the plurality of healthiness indices into one piece of general information and outputting the thus obtained general information, the processor 16 calculates, with respect to at least one of the plurality of indices, each condition as a specific numerical value and outputs such a numerical value; this makes it possible for the user to make a detailed quantitative judgment of his/her current physical condition based on the information accessible where it is outputted (for example, the display 4). This makes it easy to compare the information regarding the user's current physical condition and symptom with past records or with information of another person, and to make a specific decision on suitable measures to take according to the physical condition and symptom. For example, it becomes easy for the user to select and buy a nonprescription drug suitable for his/her physical condition and symptom, and to select and consult a medical institution suitable for his/her physical condition and symptom. In particular, the processor 16 calculates numerical values indicating conditions regarding the plurality of indices, and this makes it possible for the user to make a more detailed judgment on his/her physical condition and symptom, in comparison with a case where a numerical value is calculated only with respect to a single index.

Further, since the processor 16 performs the calculation by using the image (data) of the tongue taken by the imager 3, it doesn't take long to acquire a result in comparison with a typical physical checkup, and thus it is possible to acquire numerical values of indices quickly at a low cost. As a result, it also becomes easy for the user to check (monitor) the daily variation in his or her physical condition by using the calculated numerical values.

In particular, since the numerical value calculated by the processor 16 for each index is displayed on the display 4 or outputted from the audio output unit 7 in the form of sound, the user can immediately grasp the numerical value via the display or the sound. As a result, it is possible to securely achieve the advantages described above including, for example, easy comparison of the current information with past records or with data of another person.

Further, the user can grasp the numerical value also at an external terminal device to which the numerical value calculated by the processor 16 for each index is transmitted, and moreover, when it is a medical specialist that grasps the numerical value at the external terminal device, the medical specialist can grasp the details of the user's physical condition and symptom, and thereby provide the user with an appropriate piece of advice regarding which nonprescription drug and which medical institution to choose.

Further, the processor 16 performs calculation by using, as a chromaticity value, any of the R proportion, the G proportion, and the B proportion obtained in any of the upper region (the region R1), the middle region (the region R2), and the lower region (the region R3) of the taken image of the tongue that corresponds to each index, and thereby the processor 16 calculates a numerical value that indicates the condition of each of the indices (the systolic/diastolic blood pressures, constipation, diarrhea, physical weariness caused by fatigue, blood circulation, body temperature, bronchi, and muscle fatigue); this makes it easy for the user to grasp the specific condition regarding each index, and to make a specific decision on suitable measures to take according to the condition.

**Other Examples of Index Quantification:** Although the above description deals with an example where the proportion of the R image data, the G image data, or the B image data with respect to the reference data, which is the sum of the RGB image data, is used as the chromaticity value, the reference data need not necessarily be the sum of the RGB image data.

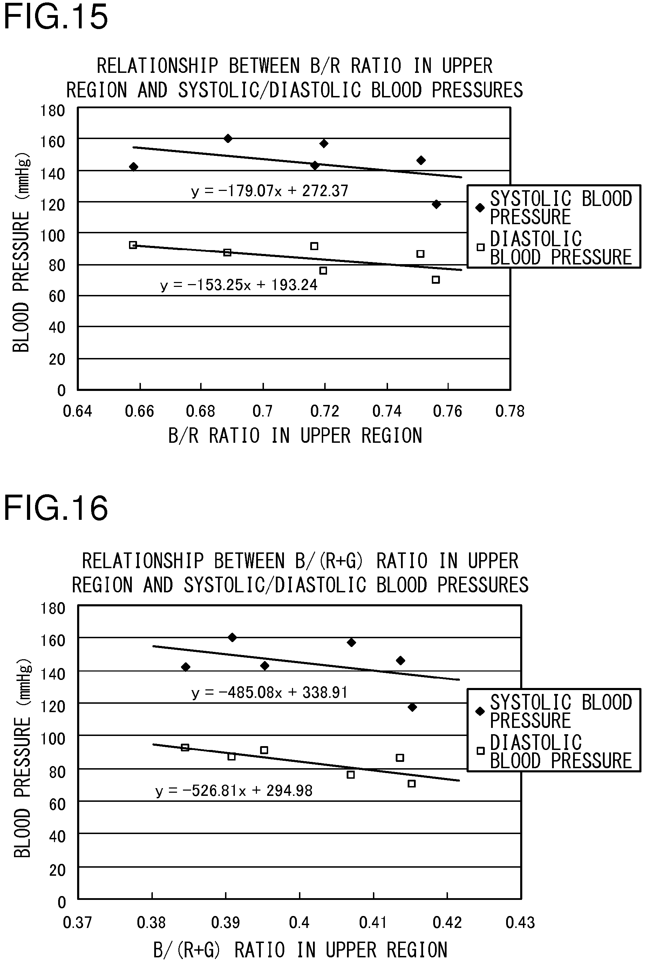

FIG. 15 is a graph illustrating a relationship between the B proportion in the upper region (the region R1) of the taken image of the tongue and systolic/diastolic blood pressures. Note that here, only the R image data is considered as the reference data, and the B proportion here is the proportion of the B image data with respect to the reference data, that is, the B proportion is a B/R ratio. From this figure, too, which shows that the B/R ratio in the upper region decreases as the systolic/diastolic blood pressures rise, it is clear that there is a correlation between them.

Obtained as a regression formula by the least squares method from the scattered data shown in FIG. 15 was the following formula:

$$\text{Systolic blood pressure: } y = -180x + 270$$

$$\text{Diastolic blood pressure: } y = -150x + 200$$

where variable x represents the B/R ratio in the upper region, and variable y represents the blood pressure (mmHg).

Thus, by using the B/R ratio, too, it is possible to calculate numerical values of the systolic/diastolic blood pressures from these regression formulae.

FIG. 16 is a graph illustrating a relationship between the B proportion in the upper region (the region R1) of the taken image of the tongue and the systolic/diastolic blood pressures. Note that here, the sum of the R image data and the G image data is considered as the reference data, and the B proportion here is the proportion of the B image data with respect to the reference data, that is, the B proportion is a B/(R + G) ratio. From this figure, too, which shows that the B/(R + G) ratio in the upper region decreases as the systolic/diastolic blood pressures rise, it is clear that there is a correlation between them.

Obtained as a regression formula by the least squares method from the scattered data shown in FIG. 16 was the following formula:

$$\text{Systolic blood pressure: } y = -500x + 340$$

$$\text{Diastolic blood pressure: } y = -530x + 300$$

where variable x represents the B/(R + G) ratio in the upper region, and variable y represents the blood pressure (mmHg).

Thus, by using the B/(R + G) ratio, too, it is possible to calculate numerical values of the systolic/diastolic blood pressures from these regression formulae.

Although the above description deals with the systolic/diastolic blood pressures as indices, but it has been found that, with respect to any of the other indices (such as the condition of constipation), too, it is possible to calculate a numerical value that indicates the condition of the index in the same manner as described above. That is, a numerical value indicating the condition of an index can be calculated also by using, as the chromaticity value, the ratio of any of the RGB image data with respect to the reference data that is not the sum of the RGB image data.

Here, however, any data with which the chromaticity value will be 1 is excluded from options of the reference data. For example, when only the R image data is considered as the reference data, the proportion of the R image data with respect to the reference data will be 1 regardless of whether the R image data is big or small, thus there is no point in using the proportion as the chromaticity value. The same is the case with respect to the proportions of the G image data and the B image data with respect to the reference data.

What can be said based on the foregoing is that the chromaticity value used to calculate an index, which is the proportion of the R image data, the proportion of the G image data, or the proportion of the B image data with respect to the reference data that includes at least any of the RGB image data, should be a value other than 1 (that is, (image data/reference data) should be a value other than R/R, G/G, and B/B).

**Others:** Although the above description deals with a case where the imaging object is the human tongue, the living body (something alive) does not necessarily have to be a human but may be any animal other than a human. For example, also with the tongue of a pet or a domestic animal, it is possible to calculate numeric values of indices through application of the method according to the present embodiment to make a judgment on the physical condition and symptom based on the thus obtained numerical values. This makes it possible to make a quick and correct judgment on poor health condition of an animal, which cannot verbally tell its physical condition to people.

An organ imaging device described herein can be said to be configured, and provides benefits, as described below.

As described herein, an organ imaging device includes an imager which images a tongue of a living body, and a processor which calculates a numerical value indicating condition regarding at least one of a plurality of healthiness indices by using a chromaticity value obtained from data of a taken image of the tongue acquired by the imager, and outputs the numerical value.

According to this configuration, the processor calculates and outputs the condition of at least one of the plurality of healthiness indices as a numerical value, and thus, for example, if the destination of the output is a display, it is possible for a user to grasp the numerical value via display performed at the display; if the destination of the output is an audio output unit, it is possible for the user to grasp the numerical value as sound from the audio output unit; and if the destination of the output is an external terminal device, it is possible for the user to grasp the numerical value at the external terminal device. This allows the user to make a detailed quantitative judgment on his/her own physical condition and symptom based on the thereby grasped numeric value of the index. As a result, it becomes easy to conduct comparison between the information regarding the user's current physical condition and symptom with the past records or with such information of another person, and to make a specific decision on suitable measures to take according to the physical condition and symptom. Further, with the configuration where the above-described numerical value is calculated through calculation performed by making use of the taken image of the tongue, it is possible to obtain the above-described numerical value at a low cost and shortly after the start of the imaging of the tongue. Thus, it also becomes easy for the user to check the daily variation in his or her physical condition by using the above-described numerical value.

The chromaticity value is preferably a value obtained in any of three upper, center, and lower regions of a center part of the taken image of the tongue in the left/right direction that corresponds to the index.

In Oriental medicine, there is known a method for diagnosing health condition and disease condition by examining the condition of the human tongue (tongue diagnosis). The color of the tongue coating, the shape (thickness) of the tongue coating, and the color of the tongue appear in chromaticity of three regions, namely, an upper region, a center region, and a lower region of the center part of the taken image of the tongue in the left/right direction. Thus, by the processor performing a calculation by using a chromaticity value obtained in at least any of the three regions, it is possible to reproduce the diagnostic method (judgment on health condition and disease symptom) of Oriental medicine. Further, by the processor performing a calculation by using a chromaticity value obtained in any of the above-described three regions that most appropriately corresponds to an index, it is possible to calculate a numerical value indicating the condition of the index with accuracy (as a numerical value approximately indicating the actual health condition).

The chromaticity value may be a value obtained as the proportion of red image data, green image data, or blue image data with respect to reference data including at least any of the red image data, the green image data, and the blue image data, and that is not 1.

When imaging is performed in a bright environment, at least any of the RGB image data increases. By using as the chromaticity value the proportion of the image data of a desired color with respect to the reference data, it is possible to reduce influence of the brightness under which imaging is performed. That is, it is possible to reduce variation in calculated numerical value of an index caused by variation in chromaticity value depending on the brightness.

It should be noted that, for example, when reference data is constituted only by the R image data, the proportion of the R image data with respect to the reference data will always be 1 regardless of whether the R image data is large or small, and thus there is no point in using the proportion as a chromaticity value, and thus such reference data is excluded from options of reference data (the above discussed advantages can be obtained when the chromaticity takes a value other than 1).

The chromaticity value may be the proportion of blue image data with respect to the reference data in the upper region of the taken image of the tongue, and the processor may calculate a numerical value of the systolic or diastolic blood pressure as one of the indices by calculation performed by using the proportion. In this case, it becomes easy for the user to specifically grasp the systolic or diastolic blood pressure from the numerical value, and to make a specific decision on measures to take against the systolic or diastolic blood pressure.

The chromaticity value may be the proportion of red image data with respect to the reference data in the upper region of the taken image of the tongue, and the processor may calculate a numerical value indicating condition of constipation as one of the indices by calculation using the proportion. In this case, it becomes easy for the user to specifically grasp the condition of constipation from the numerical value, and to make a specific decision on measures to take against the constipation.

The chromaticity value may be the proportion of green image data with respect to the reference data in the center region of the taken image of the tongue, and the processor may calculate a numerical value indicating condition of diarrhea as one of the indices by calculation using the proportion. In this case, it becomes easy for the user to specifically grasp the condition of diarrhea from the numerical value, and to make a specific decision on measures to take against the diarrhea.

The chromaticity value may be the proportion of green image data with respect to the reference data in the center region of the taken image of the tongue, and the processor may calculate a numerical value indicating condition of physical weariness caused by fatigue as one of the indices by calculation using the proportion. In this case, it becomes easy for the user to specifically grasp the condition of physical weariness caused by fatigue from the numerical value, and to make a specific decision on measures to take against the physical weariness.

The chromaticity value may be the proportion of blue image data with respect to the reference data in the center region of the taken image of the tongue, and the processor may calculate a numerical value indicating condition of blood circulation as one of the indices by calculation using the proportion. In this case, it becomes easy for the user to specifically grasp the condition of blood circulation from the numerical value, and to make a specific decision on measures to take against the condition of blood circulation.

The chromaticity value may be the proportion of red image data with respect to the reference data in the lower region of the taken image of the tongue, and the processor may calculate a numerical value of body temperature as one of the indices by calculation using the proportion. In this case, it becomes easy for the user to specifically grasp the body temperature from the numerical value, and to make a specific decision on measures to take against the body temperature.

The chromaticity value may be the proportion of green image data with respect to the reference data in the lower region of the taken image of the tongue, and the processor may calculate a numerical value indicating condition of bronchi as one of the indices by calculation using the proportion. In this case, it becomes easy for the user to specifically grasp the condition of his/her bronchi from the numerical value, and to make a specific decision on measures to take against the condition of his/her bronchi.

The chromaticity value may be the proportion of green image data with respect to the reference data in the lower region of the taken image of the tongue, and the processor may calculate a numerical value indicating condition of muscle fatigue as one of the indices by calculation using the proportion. In this case, it becomes easy for the user to specifically grasp the condition of muscle fatigue from the numerical value, and to make a specific decision on measures to take against the muscle fatigue.

The reference data may be a sum of red, green, and blue image data. When imaging is performed in a bright environment, the red image data, the green image data, and the blue image data each increases, and thus, by considering the proportion of image data of a desired color with respect to the reference data which is a sum of the RGB image data, it is possible to securely reduce the influence of brightness at the time of imaging.

The organ imaging device may further include an output unit that displays, or outputs in a form of sound, the numerical value outputted from the processor. In this case, the user can instantly grasp the numerical value of each index through the display at, or sound outputted from, the output unit, which makes it even easier to monitor the daily variation in physical condition and to conduct comparison with past records or data of another person, and to make a specific decision on suitable measures to take according to the physical condition and symptom.

**Industrial Applicability**

The present invention is applicable to a device that images the tongue as an organ of a living body and extracts from the taken image such information as is necessary for a healthiness checkup.

**List of Reference Signs**

1       organ imaging device
3       imager
4       display (output unit)
7       audio output unit (output unit)
16      processor
Q       contour line
R1      region (upper region)
R2      region (center region)
R3      region (lower region)

**Claims**

1.   An organ imaging device comprising:

an imager which images a tongue of a living body; and
a processor which calculates a numerical value indicating condition regarding at least one of a plurality of healthiness indices by using a chromaticity value obtained from data of a taken image of the tongue acquired by the imager, and outputs the numerical value.

2.   The organ imaging device according to claim 1, wherein
the chromaticity value is a value obtained in any of three upper, center, and lower regions in a center part of the taken image of the tongue in a left/right direction that corresponds to each of the indices.

3.   The organ imaging device according to claim 2, wherein
the chromaticity value is proportion of red image data, proportion of green image data, or proportion of blue image data with respect to reference data including at least any of the red image data, the green image data, and the blue image data, and the chromaticity value is a value other than 1.

4.   The organ imaging device according to claim 3, wherein
the chromaticity value is proportion of blue image data with respect to the reference data in the upper region of the taken image of the tongue; and
the processor calculates a numerical value of a systolic or diastolic blood pressure as one of the indices by calculation using the proportion.

5.   The imaging device according to claim 3 or 4, wherein
the chromaticity value is proportion of red image data with respect to the reference data in the upper region of the taken image of the tongue; and
the processor calculates a numerical value indicating condition of constipation as one of the indices by calculation using the proportion.

6.   The organ imaging device according to any one of claims 3 to 5, wherein
the chromaticity value is proportion of green image data with respect to the reference data in the center region of the taken image of the tongue; and
the processor calculates a numerical value indicating condition of diarrhea as one of the indices by calculation using the proportion.

7.   The organ imaging device according to any one of claims 3 to 6, wherein
the chromaticity value is proportion of green image data with respect to the reference data in the center region of the taken image of the tongue; and
the processor calculates a numerical value indicating condition of physical weariness caused by fatigue as one of the indices by calculation using the proportion.

8.   The organ imaging device according to any one of claims 3 to 7, wherein
the chromaticity value is proportion of blue image data with respect to the reference data in the center region of the taken image of the tongue; and
the processor calculates a numerical value indicating condition of blood circulation as one of the indices by calculation using the proportion.

9.   The organ imaging device according to any one of claims 3 to 8, wherein
the chromaticity value is proportion of red image data with respect to the reference data in the lower region of the taken image of the tongue; and
the processor calculates a numerical value of body temperature as one of the indices by calculation using the proportion.

10. The organ imaging device according to any one of claims 3 to 9, wherein

the chromaticity value is proportion of green image data with respect to the reference data in the lower region of the taken image of the tongue; and the processor calculates a numerical value indicating condition of bronchi as one of the indices by calculation using the proportion.

11. The organ imaging device according to any one of claims 3 to 10, wherein the chromaticity value is proportion of green image data with respect to the reference data in the lower region of the taken image of the tongue; and the processor calculates a numerical value indicating condition of muscle fatigue as one of the indices by calculation using the proportion.

12. The organ imaging device according to any one of claims 3 to 11, wherein the reference data is a sum of red, green, and blue image data.

13. The organ imaging device according to any one of claims 1 to 12, further comprising an output unit that displays, or outputs in a form of sound, the numerical value outputted from the processor.

FIG.1

# FIG.2

1

OVERALL CONTROLLER — 20

ILLUMINATION CONTROLLER — 11 → ILLUMINATOR — 2

IMAGING CONTROLLER — 12 → IMAGER — 3

DISPLAY CONTROLLER — 13 → DISPLAY — 4

OPERATION CONTROLLER — 14 → OPERATION UNIT — 5

IMAGE PROCESSING UNIT — 15

PROCESSOR — 16

STORAGE — 17

COMMUNICATION CONTROLLER — 18 → COMMUNICATOR — 6

AUDIO OUTPUT CONTROLLER — 19 → AUDIO OUTPUT UNIT — 7

FIG.3

# FIG.4

TAKEN IMAGE

4

EDGE EXTRACTION FILTER

| | −1 | |
|---|---|---|
| −1 | 4 | −1 |
| | −1 | |

EXTRACTED CONTOUR
LINE

Q

# FIG.5

# FIG.6

RELATIONSHIP BETWEEN B PROPORTION IN
UPPER REGION AND SYSTOLIC
/DIASTOLIC BLOOD PRESSURES

$y = -944.54x + 414.69$

$y = -1033x + 379.35$

SYSTOLIC BLOOD
PRESSURE
DIASTOLIC
BLOOD PRESSURE

BLOOD PRESSURE (mmHg)

B PROPORTION IN UPPER REGION

# FIG.7

RELATIONSHIP BETWEEN R PROPORTION IN
UPPER REGION AND CONSTIPATION

$y = 81.428x - 30.78$

CONSTIPATION RANKS

R PROPORTION IN UPPER REGION

# FIG.8

RELATIONSHIP BETWEEN G PROPORTION IN
CENTER REGION AND CONDITION OF DIARRHEA

$y = 193.98x - 59.577$

DIARRHEA RANKS

G PROPORTION IN CENTER REGION

## FIG.9

RELATIONSHIP BETWEEN G PROPORTION IN
CENTER REGION AND PHYSICAL WEARINESS

$y = -176.11x + 55.937$

PHYSICAL WEARINESS RANKS

G PROPORTION IN CENTER REGION

## FIG.10

RELATIONSHIP BETWEEN B PROPORTION IN CENTER
REGION AND UNDER-EYE DARK CIRCLES

$y = 29.476x - 7.7311$

UNDER-EYE DARK CIRCLES RANKS

B PROPORTION IN CENTER REGION

# FIG.11

RELATIONSHIP BETWEEN R PROPORTION IN LOWER
REGION AND BODY TEMPERATURE

y = 13.653x + 30.792

# FIG.12

RELATIONSHIP BETWEEN G PROPORTION IN LOWER
REGION AND SORE THROAT

y = −76.746x + 22.947

# FIG.13

### RELATIONSHIP BETWEEN G PROPORTION IN LOWER REGION AND CONDITION OF LEG CRAMP

$y = 170.77x - 49.349$

LEG CRAMP RANKS (vertical axis)

G PROPORTION IN LOWER REGION (horizontal axis)

# FIG.14

START

TURN ON ILLUMINATION — S1

SET IMAGING CONDITIONS — S2

PERFORM IMAGING — S3

EXTRACT CONTOUR LINE — S4

SET REGIONS — S5

CALCULATE CHROMATICITY VALUES (RGB PROPORTIONS) — S6

CALCULATE NUMERICAL VALUE OF EACH INDEX — S7

DISPLAY, RECORD, TRANSMIT — S8

END

## FIG.15

RELATIONSHIP BETWEEN B/R RATIO IN UPPER
REGION AND SYSTOLIC/DIASTOLIC BLOOD PRESSURES

$y = -179.07x + 272.37$

$y = -153.25x + 193.24$

SYSTOLIC BLOOD PRESSURE

DIASTOLIC BLOOD PRESSURE

B/R RATIO IN UPPER REGION

## FIG.16

RELATIONSHIP BETWEEN B/(R+G) RATIO IN UPPER
REGION AND SYSTOLIC/DIASTOLIC BLOOD PRESSURES

$y = -485.08x + 338.91$

$y = -526.81x + 294.98$

SYSTOLIC BLOOD PRESSURE

DIASTOLIC BLOOD PRESSURE

B/(R+G) RATIO IN UPPER REGION

## INTERNATIONAL SEARCH REPORT

| International application No. |
|---|
| PCT/JP2014/082393 |

### A. CLASSIFICATION OF SUBJECT MATTER
*A61B5/00*(2006.01)i

According to International Patent Classification (IPC) or to both national classification and IPC

### B. FIELDS SEARCHED

Minimum documentation searched (classification system followed by classification symbols)
A61B5/00

Documentation searched other than minimum documentation to the extent that such documents are included in the fields searched
Jitsuyo Shinan Koho        1922–1996    Jitsuyo Shinan Toroku Koho   1996–2015
Kokai Jitsuyo Shinan Koho    1971–2015    Toroku Jitsuyo Shinan Koho   1994–2015

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2004-113581 A  (Choyo Corp.),<br>15 April 2004 (15.04.2004),<br>paragraphs [0021] to [0023]<br>(Family: none) | 1,13<br>2-3,12<br>4-11 |
| Y<br>A | JP 2006-149679 A  (Konica Minolta Holdings,<br>Inc.),<br>15 June 2006 (15.06.2006),<br>paragraphs [0066] to [0067]; fig. 9<br>(Family: none) | 2,3,12<br>1,4-11,13 |
| A | JP 2006-166990 A  (Olympus Corp.),<br>29 June 2006 (29.06.2006),<br>paragraph [0052]<br>& US 2008/0292154 A1    & EP 1842481 A1<br>& WO 2006/062163 A1    & KR 10-2007-0085875 A | 1-13 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    10 February 2015 (10.02.15) | Date of mailing of the international search report<br>    24 February 2015 (24.02.15) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (July 2009)

**INTERNATIONAL SEARCH REPORT**

| International application No. |
|---|
| PCT/JP2014/082393 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2004-209245 A  (Samsung Electronics Co., Ltd.), 29 July 2004 (29.07.2004), entire text; all drawings & US 2004/0151379 A1     & EP 1450287 A2 & KR 10-0506085 B1       & CN 1512451 A | 1-13 |
| A | CN 102509312 B  (Harbin Institute of Technology), 02 October 2013 (02.10.2013), entire text; all drawings (Family: none) | 1-13 |

Form PCT/ISA/210 (continuation of second sheet) (July 2009)

**EP 3 100 672 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4487535 B
- JP 4649965 B